(19) Europäisches Patentamt
European Patent Office
Office européen des brevets

(11) Numéro de publication : 0 085 667 B1

(12) **FASCICULE DE BREVET EUROPÉEN**

(45) Date de publication du fascicule du brevet : 07.05.86

(51) Int. Cl.⁴ : C 12 Q 1/34, G 01 N 33/04

(21) Numéro de dépôt : 83870007.8

(22) Date de dépôt : 31.01.83

# (54) Procédé enzymatique pour la détermination d'antibiotiques à noyau bêta-lactame.

(30) Priorité : 01.02.82 GB 8202790

(43) Date de publication de la demande : 10.08.83 Bulletin 83/32

(45) Mention de la délivrance du brevet : 07.05.86 Bulletin 86/19

(84) Etats contractants désignés : AT BE CH DE FR GB IT LI LU NL SE

(56) Documents cités :
FR-A- 2 293 489
ANTIMICROBIAL AGENTS AND CHEMOTHERAPY, vol. 18, no. 4, octobre 1980, pages 506-510 J.M. FRERE et al.: "Enzymatic method for rapid and sensitive determination of beta-lactam antibiotics"
CHEMICAL ABSTRACTS, vol. 90, no. 25, 18 juin 1979, page 227, colonne 2, no. 199525m, Columbus, Ohio, USA J.M. FRERE et al.: "The exocellular DD-carboxypeptidase-endopeptidase of streptomyces albus G. interaction with beta-lactam antibiotics"
CHEMICAL ABSTRACTS, vol. 85, no. 23, 6 décembre 1976, page 180, colonne 1, abrégé no. 173261u, Columbus, Ohio, USA A. MARQUET et al.: "Membrane-bound DD-carboxypeptidase and transpeptidase activities from Bacillus megaterium KM et pH7. General properties, substrate specificity and inhibition by beta-lactam antibiotics"
CHEMICAL ABSTRACTS, vol. 87, no. 7, 15 août 1977, page 73, colonne 1, abrégé no. 48470b, Columbus, Ohio, USA J. COYETTE et al.: "Interactions between beta-lactam antibiotics and isolated membranes of streptococcus faecalis ATCC 9790"
CHEMICAL ABSTRACTS, vol. 88, no. 5, 30 janvier 1978, pages 187, 188, abrégé no. 33780z, Columbus, Ohio, USA S.S. TRAPEZNIKOVA et al : " Interaction between immobilized carboxypeptidase N and its natural inhibitor from human blood plasma ".

(73) Titulaire : U C B
326, Avenue Louise
B-1050 Bruxelles (BE)

(72) Inventeur : Degelaen, Jacques
14, Avenue des Taillis
B-1488 Genappe (BE)
Inventeur : Loffet, Albert
14, Rue Mathias
B-1440 Braine-le-Chateau (BE)
Inventeur : Durieux, Jean-Pierre
107, Rue Notre-Dame au Bois
B-1440 Braine-le-Chateau (BE)

(74) Mandataire : Vanderborght, Henri et al
U C B, S.A. Département D.T.B. 326, avenue Louise - Bte 7
B-1050 Bruxelles (BE)

EP 0 085 667 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention se rapporte à un nouveau procédé enzymatique, rapide et sensible pour la détermination d'antibiotiques à noyau bêta-lactame dans un liquide biologique. Elle concerne également une trousse d'essai utilisable pour la mise en œuvre de ce procédé.

A l'heure actuelle, les antibiotiques sont très largement utilisés non seulement comme agents thérapeutiques dans le traitement des maladies infectieuses provoquées par les bactéries, mais également comme agents de conservation des aliments et comme additifs dans la nourriture des animaux pour stimuler la croissance. La nécessité se fait donc de plus en plus sentir de pouvoir détecter la présence d'antibiotiques, même en de très faibles concentrations, dans des liquides biologiques complexes, comme le lait, l'urine, le sang, le sérum, la salive, les extraits de viande, les liquides de fermentation, etc.

Le cas de la production du lait en est un exemple. Il est bien connu en effet d'utiliser les pénicillines pour traiter certaines maladies infectieuses du bétail producteur de lait, comme par exemple la mastite. Or, pour des raisons médicales évidentes, le lait destiné à la consommation humaine doit, en principe, être exempt de toute trace d'antibiotiques. Par ailleurs, des concentrations en pénicilline de 0,005 U.I./ml ou moins peuvent avoir des incidences néfastes lors de la fabrication de produits dérivés du lait, comme le fromage, le yaourt, etc.

Il s'avère donc qu'il est nécessaire de pouvoir déterminer, rapidement et de manière précise, la concentration des pénicillines dans le lait produit par le bétail et ce, de préférence, directement sur place à la ferme.

Il existe depuis longtemps des procédés microbiologiques qui permettent de déterminer des concentrations relativement faibles d'antibiotiques bêta-lactames dans des liquides biologiques. Ces procédés sont basés sur la mesure de l'inhibition de la croissance de microorganismes sensibles aux antibiotiques, en présence d'un échantillon du liquide biologique. Cependant, ces procédés exigent beaucoup de temps et une grande technicité ; dans le meilleur des cas, la durée nécessaire pour obtenir un résultat est d'environ 2 à 3 heures, ce qui n'est pas admissible en pratique.

Plus récemment on a proposé également un procédé microbiologique rapide pour détecter la présence d'antibiotiques dans un liquide biologique, en particulier dans le lait (cf. brevet américain nº 4.239.852).

Selon cette méthode, l'échantillon du liquide à examiner est incubé, d'une part avec des cellules ou parties de cellules d'un microorganisme très sensible aux antibiotiques, en particulier Bacillus stearothermophilus et d'autre part, avec un antibiotique marqué par un élément radioactif ou par une enzyme. Au cours de l'incubation, l'antibiotique éventuellement présent dans l'échantillon et l'antibiotique marqué entrent en compétition pour se fixer sur les sites récepteurs des cellules ou parties de cellules. Ensuite, on détermine la quantité d'antibiotique marqué qui a été fixée sur les cellules ou parties de cellules ; ceci fournit une indication de la présence (ou non) d'antibiotiques, étant donné que la quantité d'antibiotique marqué fixée est inversement proportionnelle à la concentration d'antibiotique dans l'échantillon.

D'après l'auteur de ce brevet, ce procédé permettrait de détecter des concentrations en antibiotique aussi faibles que 0,01 U.I./ml, voire même de 0,001 U.I./ml dans le lait en un peu moins de 15 minutes.

Mais, l'inconvénient majeur de ce procédé réside dans le fait que, pour atteindre ce résultat, il faut obligatoirement avoir recours à un antibiotique marqué par un élément radioactif ($^{14}C$ ou $^{125}I$), qu'il faut doser à l'aide d'un appareil spécial, comme par exemple un compteur à scintillation. De plus, la manipulation de produits radioactifs, même en très faibles quantités, n'est pas totalement exempte de danger pour la personne qui effectue l'analyse.

Il est vrai que dans l'exemple 2 de ce brevet on décrit une variante de ce procédé selon laquelle on utilise un antibotique marqué par une enzyme et dans laquelle on détermine la quantité d'antibiotique marqué par une méthode colorimétrique visuelle. Mais, cette variante ne permet de détecter que s'il y a plus (ou moins) de 0,05 U.I./ml de pénicilline dans l'échantillon de lait. Ce procédé est donc nettement moins sensible et par conséquent beaucoup moins intéressant.

Dans un article récent, on décrit un procédé enzymatique permettant de déterminer de faibles concentrations d'antibiotiques bêta-lactames dans le sérum humain et dans le lait (J.-M. FRERE, D. KLEIN et J.-M. GHUYSEN, Antimicrobial Agents and Chemotherapy, 18, (1980, nº 4), p. 506-510).

Ce procédé (désigné dans la description par « procédé de J.-M. FRERE ») est beaucoup plus intéressant parce qu'il ne nécessite pas le recours à des produits radioactifs requérant des appareils de mesure sophistiqués, tout en étant d'une très grande rapidité et d'une précision remarquable. Ce procédé est basé sur l'utilisation d'une enzyme spécifique, en l'occurrence de la D-alanyl-D-alanine-carboxypeptidase exocellulaire soluble, qui est produite par Actinomadura R 39 (précédemment dénommée Streptomyces R 39). Dans la description qui va suivre, cette enzyme sera désignée par « enzyme R 39 ». Comme son nom l'indique, l'enzyme R 39 possède une activité spécifique d'hydrolyse des groupements terminaux D-alanyl-D-alanine de divers peptides. A titre d'exemple, le tripeptide $N^{alpha}$, $N^{epsilon}$-diacétyl-L-lysyl-D-alanyl-D-alanine subit une réaction d'hydrolyse sous l'influence catalytique de l'enzyme R 39, selon l'équation :

$$Ac_2\text{-L-Lys-D-Ala-D-Ala} + H_2O \xrightarrow{\text{enzyme}} Ac_2\text{-L-Lys-D-Ala} + \text{D-alanine.}$$

Une autre caractéristique importante de l'enzyme R 39 réside dans le fait qu'elle réagit avec les antibiotiques à noyau bêta-lactame pour former très rapidement un complexe enzyme-antibiotique équimoléculaire, inactif et sensiblement irréversible.

Dans le procédé de J.-M. FRERE, on met à profit ces propriétés de l'enzyme R 39 pour déterminer de très faibles concentrations d'antibiotiques bêta-lactames. Ce procédé comporte trois stades essentiels. Dans un premier stade, un volume déterminé d'un échantillon du liquide à examiner est incubé avec une quantité déterminée de l'enzyme R 39. L'incubation est menée dans des conditions permettant à l'antibiotique bêta-lactame éventuellement présent dans l'échantillon de réagir avec l'enzyme pour former un complexe enzyme-antibiotique équimoléculaire, inactif et sensiblement irréversible.

Dans un deuxième stade, une quantité déterminée de substrat, par exemple la $N^{alpha}$, $N^{epsilon}$-diacétyl-L-lysyl-D-alanyl-D-alanine, est incubée avec le produit obtenu au premier stade dans des conditions permettant l'hydrolyse du substrat par l'enzyme pour former une quantité de D-alanine correspondant à l'activité enzymatique résiduelle de l'enzyme R 39 qui n'a pas été complexée avec l'antibiotique dans le premier stade.

Dans un troisième stade, on détermine la quantité de D-alanine ainsi formée. L'homme de métier comprendra aisément qu'en fonction de la quantité d'antibiotique présent dans l'échantillon une quantité correspondante d'enzyme R 39 sera inactivée au premier stade et que la quantité de D-alanine formée au deuxième stade (qui dépend évidemment de l'activité résiduelle de l'enzyme) est donc inversement proportionnelle à la quantité d'antibiotique présente dans l'échantillon. Si, par exemple, l'échantillon est exempt d'antibiotique, l'enzyme R 39 n'est pas inactivée et l'on trouve une quantité de D-alanine qui correspond à l'activité totale de l'enzyme R 39 mise en œuvre. Par contre, si l'échantillon contient une quantité molaire en antibotique égale ou supérieure à la quantité molaire d'enzyme R 39 mise en œuvre, celle-ci est complètement inactivée par l'antibiotique et il n'y a pas de D-alanine formée. Entre ces deux situations extrêmes, on trouve une quantité de D-alanine qui correspond au pourcentage d'activité résiduelle de l'enzyme R 39. En d'autres termes, la quantité de D-alanine formée fournit une indication quantitative précise de la concentration en antibiotique présente dans l'échantillon soumis à l'examen.

Dans le procédé de J.-M. FRERE, on détermine cette quantité de D-alanine par une méthode enzymatique. Celle-ci repose sur deux réactions enzymatiques couplées. Dans la première réaction, la D-alanine est oxydée en acide pyruvique à l'aide d'une D-aminoacide-oxydase (ensemble avec son coenzyme, le flavine-adénine-dinucléotide) ; il se forme en même temps une quantité correspondante de peroxyde d'hydrogène à partir de l'oxygène de l'air. Dans la deuxième réaction, le peroxyde d'hydrogène formé est utilisé pour oxyder l'o-dianisidine à l'aide d'une peroxydase.

C'est pourquoi, au troisième stade du procédé de J.-M. FRERE, on incube le mélange obtenu au deuxième stade avec un ensemble de réactifs comprenant une D-aminoacide-oxydase, son coenzyme (le flavine-adénine-dinucléotide ; FAD), une peroxydase et l'o-dianisidine. Comme la forme oxydée de l'o-dianisidine est colorée, au terme de cette incubation il se produit une coloration brune, dont l'intensité est fonction de la quantité de D-alanine.

Ceci permet donc de déterminer la quantité de D-alanine par une méthode colorimétrique, soit visuellement, soit par mesure de la densité optique au spectrophotomètre ($\lambda$ max = 460 nm).

En préparant une série d'échantillons d'une concentration connue en antibiotique et en appliquant ce procédé, on peut ainsi établir une courbe d'étalonnage qui relie le pourcentage d'activité enzymatique résiduelle de l'enzyme R 39 à la concentration en antibiotique.

Pour obtenir une indication quantitative de la concentration en antibiotique d'un échantillon, on procède alors exactement de la même manière et on détermine la concentration en antibiotique en se reportant à cette courbe d'étalonnage.

Cette évaluation quantitative nécessite évidemment le recours à un spectrophotomètre.

Mais, pour déterminer si la concentration en antibiotique dépasse ou non une certaine valeur critique, il n'est pas nécessaire de recourir à un spectrophotomètre ou un autre appareil similaire de mesure. Il suffit de connaître au préalable à quelle concentration critique en antibiotique l'activité de l'enzyme R 39 est totalement supprimée ou, autrement dit, à quelle concentration en antibiotique il ne se forme pas de D-alanine et, par conséquent, il ne se produit pas de coloration au terme de la dernière incubation. Connaissant cette concentration critique, il est évident que l'on peut, par une simple inspection visuelle du résultat de la détermination, juger si oui ou non un échantillon donné contient une concentration en antibiotique moins grande ou plus grande que ladite concentration critique. On voit donc que par ce procédé on peut, sans disposer d'un appareil particulier, obtenir rapidement et de manière précise une indication de la concentration en antibiotique dans le lait.

En outre, ce procédé permet de déterminer des concentrations relativement faibles en antibiotique bêta-lactame dans le lait et dans le sérum humain. Ainsi par exemple, en partant d'échantillons de lait d'un volume d'environ 20 µl et en incubant ceux-ci avec 3 picomoles d'enzyme R 39, il est possible de déterminer, quantitativement (en utilisant un spectrophotomètre), des concentrations en pénicilline aussi faibles que 0,02 U.I./ml de lait et, qualitativement, par la méthode visuelle décrite plus haut, des concentrations de plus de 0,09 U.I./ml de lait en moins d'une heure.

Cependant, le procédé de J.-M. FRERE présente divers inconvénients importants.

En premier lieu, pour la détermination d'antibiotiques dans les liquides biologiques comme le lait, le sérum, etc., il est indispensable d'éliminer au préalable des échantillons soumis à la détermination les substances qui peuvent perturber le dosage colorimétrique. Dans le cas du lait par exemple, il faut d'abord précipiter les protéines avec de l'acide citrique ou de la présure, puis filtrer l'échantillon ainsi traité de manière à obtenir un filtrat clair qui ne peut plus perturber le dosage colorimétrique. Cette opération de précipitation des protéines est complexe et fastidieuse ce qui fait que le procédé peut difficilement être exécuté sur le lieu de production du lait par des personnes non spécialisées.

En second lieu, la sensibilité du procédé de J.-M. FRERE, en particulier dans le cas de liquides biologiques (lait, salive, sérum, etc.), est insuffisante. Il est vrai que l'on pourrait augmenter cette sensibilité en diminuant la quantité d'enzyme R 39 mise en œuvre. En effet, si on utilisait par exemple 0,3 picomole d'enzyme au lieu de 3 picomoles, on pourrait, théoriquement, déterminer des concentrations en antibiotique qui sont dix fois plus faibles. Toutefois, lorsqu'on diminue la quantité d'enzyme, il faut aussi augmenter dans les mêmes proportions d'une part la durée de réaction entre l'antibiotique et l'enzyme R 39 et, d'autre part, le temps d'hydrolyse du substrat. Cela signifie qu'aussi bien le premier que le deuxième stade du procédé durent environ dix fois plus longtemps.

On supprimerait ainsi un des avantages primordiaux du procédé de J.-M. FRERE, à savoir sa rapidité, ce qui est inadmissible.

Par ailleurs, l'on pourrait également tenter d'augmenter la sensibilité, en augmentant le volume de l'échantillon qu'il faut incuber avec l'enzyme R 39. En effet, si on utilisait par exemple un échantillon d'un volume de 200 μl au lieu de 20 μl, on pourrait théoriquement déterminer des concentrations en antibotique qui sont dix fois plus faibles. En outre, dans ce cas, il faudrait seulement augmenter la durée de réaction entre l'antibiotique et l'enzyme R 39, c'est-à-dire uniquement le temps du premier stade du procédé.

Malheureusement, il n'est pas possible d'augmenter de cette manière la sensibilité du procédé de J.-M. FRERE, en particulier dans le cas de liquides complexes d'origine biologique. On a constaté en effet qu'il est impossible de réaliser une détermination convenable lorsque le volume de l'échantillon de liquide biologique dépasse un certain volume critique, qui varie en fonction de la nature du liquide biologique. Ainsi, dans le cas du lait, lorsque le volume de l'échantillon que l'on soumet à l'incubation dépasse un volume de ± 20 μl, on observe que la quantité de o-dianisidine oxydée formée est fortement réduite. Il en est de même pour le sérum, lorsque le volume de l'échantillon incubé avec l'enzyme R 39 dépasse ± 50 μl.

Enfin, dans le cas de l'urine, on ne détecte aucune activité enzymatique, même en utilisant des volumes d'échantillon aussi faibles que 10 μl. Il est donc impossible d'utiliser ce procédé pour la détermination d'antibiotiques dans l'urine.

On suppose que les liquides biologiques renferment des substances qui inhibent l'action des enzymes utilisées dans le procédé, d'où l'impossibilité d'utiliser de grands volumes d'échantillons.

C'est pourquoi, la demanderesse a mis au point un nouveau procédé enzymatique pour la détermination d'antibiotiques à noyau bêta-lactame dans un liquide biologique, qui est plus perfectionné, en ce sens qu'il ne présente pas les divers inconvénients des procédés de l'état de la technique, rappelés ci-dessus.

La demanderesse vient en effet de découvrir présentement qu'il est possible de supprimer les divers inconvénients du procédé de J.-M. FRERE, en immobilisant l'enzyme R 39 sur un support insoluble dans l'eau, en particulier sur une résine de poly(N,N-diméthylacrylamide).

La présente invention a donc pour objet un procédé enzymatique amélioré pour la détermination d'antibiotiques à noyau bêta-lactame dans un liquide biologique et comprenant les stades suivants :

1) l'incubation dudit liquide avec la D-alanyl-D-alanine-carboxypeptidase soluble produite par Actinomadura R 39, cette incubation étant menée dans des conditions permettant à l'antibiotique bêta-lactame éventuellement présent dans ledit liquide de réagir avec l'enzyme pour former un complexe enzyme-antibiotique équimoléculaire, inactif et sensiblement irréversible ;

2) l'incubation du produit obtenu à la fin du stade (1) avec une solution de substrat dans des conditions permettant l'hydrolyse dudit substrat par l'enzyme pour former une quantité de D-alanine correspondant à l'activité enzymatique résiduelle ;

3) la détermination de la quantité de D-alanine formée au stade (2) ;

4) la comparaison de la détermination du stade (3) avec un étalon pour obtenir la concentation de l'antibiotique dans le liquide biologique,

et qui est caractérisé en ce que l'enzyme mise en œuvre est immobilisée sur un support insoluble dans l'eau et en ce que cette enzyme immobilisée est séparée à partir du liquide biologique et lavée, avant d'être incubée avec la solution de substrat au stade (2).

A la différence du procédé de J.-M. FRERE, le procédé conforme à l'invention permet de faire une détermination directement sur le liquide biologique tel quel. Il n'est pas nécessaire d'enlever au préalable des échantillons, soumis à la détermination, les substances qui peuvent perturber le dosage colorimétrique.

En effet, comme l'enzyme R 39 est immobilisée sur un support insoluble dans l'eau, après sa réaction avec l'antibiotique éventuellement présent dans le liquide biologique, cette enzyme immobilisée peut être séparée facilement du liquide biologique par une simple filtration et lavée. Par conséquent, au cours des

opérations ultérieures, il n'y a plus aucune possibilité d'interférence du liquide biologique, parce qu'il est déjà éliminé au terme du stade (1) du procédé.

On supprime ainsi un des inconvénients majeurs du procédé de J.-M. FRERE.

En outre, on a constaté avec surprise qu'en appliquant le procédé conforme à l'invention, on pouvait faire d'excellentes déterminations sur des volumes d'échantillon de liquide biologique compris entre 200 µl et 5 ml, sans la moindre difficulté.

Ceci revêt une importance capitale. En effet, comme il a été expliqué plus haut, en augmentant le volume de l'échantillon qu'il faut incuber avec l'enzyme R 39, il est possible d'augmenter la sensibilité du précédé. Or, un des inconvénients du procédé de J.-M. FRERE réside dans le fait que l'on ne peut pas dépasser un volume critique (de l'ordre de 20 µl pour le lait et de 50 µl pour le sérum). Comme cet inconvénient est éliminé par le procédé conforme à l'invention, il est évident qu'à la différence de celui de J.-M. FRERE, le procédé de l'invention permet d'obtenir une sensibilité beaucoup plus grande dans la détermination de liquides biologiques comme le lait, le sérum, etc. Comme on le montrera plus loin dans les exemples de réalisation, le procédé conforme à la présente invention permet ainsi, au départ d'échantillons de lait de 1 ml, de déterminer visuellement et avec certitude, des concentrations en pénicilline G de plus de 0,002 U.I./ml (1,2 ng/ml) de lait et, à l'aide d'un spectrophotomètre, des concentrations aussi faibles que 0,000 5 U.I./ml (0,3 ng/ml) de lait en un temps inférieur à une heure. A titre de comparaison, rappelons que le procédé décrit dans le brevet américain 4.239.852 permet de détecter des concentrations de l'ordre de 0,01 à 0,001 U.I./ml de lait, à la condition toutefois d'utilser des produits radioactifs et un compteur à scintillation.

En outre, on a constaté que le procédé conforme à la présente invention peut être appliqué avec succès, non seulement pour la détermination d'antibiotique dans le lait et le sérum, mais également dans d'autres liquides biologiques complexes comme par exemple l'urine, le sang, la salive, les extraits de viande, des liquides de fermentation, etc. Vu sous cet angle, le procédé de l'invention présente donc un progrès considérable par rapport à celui de J.-M. FRERE.

L'avantage primordial du procédé de l'invention est donc qu'il permet de détecter rapidement de très faibles concentrations en antibiotique bêta-lactame, par exemple de l'ordre de 0,002 U.I./ml, dans des liquides biologiques les plus divers, sans devoir recourir à un appareil particulier d'analyse.

Les antibiotiques, dont on peut déterminer la concentration en utilisant le procédé conforme à l'invention, appartiennent au groupe d'antibiotiques qui sont caractérisés par la présence d'un noyau bêta-lactame dans leur molécule, c'est-à-dire en principe toutes les pénicillines et les céphalosporines. A titre d'exemple de pénicillines, on peut citer la benzylpénicilline (pénicilline G), l'ampicilline, la phénoxyméthylpénicilline, la carbénicilline, la méthicilline, l'oxacilline, la cloxacilline, etc. A titre d'exemple de céphalosporines, on peut citer la céphalosporine C, la céfaloglycine, la céfalothine, la céfalexine, etc.

Des résultats particulièrement favorables ont été obtenus avec la pénicilline G.

Au stade (1) du procédé conforme à l'invention, un volume déterminé d'un échantillon du liquide biologique est incubé avec une quantité déterminée de l'enzyme R 39, immobilisée sur un support insoluble dans l'eau.

Comme il a été expliqué plus haut, grâce à l'immobilisation de l'enzyme R 39 sur un support insoluble dans l'eau, il est possible de travailler avec de très grands volumes d'échantillon. En augmentant le volume de l'échantillon, on augmente parallèlement la sensibilité du procédé. Par exemple, en doublant le volume de l'échantillon, la sensibilité est doublée, en triplant le volume de l'échantillon, la sensibilité est triplée, etc. On peut donc choisir le volume de l'échantillon en fonction de la sensibilité désirée. Dans le cas du lait par exemple, on pourra adapter la sensibilité du procédé aux normes fixées par la législation du pays où il est utilisé, ou encore aux exigences de l'industrie laitière.

Il est vrai que l'on peut également obtenir le même effet en diminuant la quantité d'enzyme R 39. Toutefois, dans ce cas, il faut augmenter proportionnellement non seulement la durée du stade (1), mais également la durée du stade (2) du procédé, alors qu'en augmentant le volume de l'échantillon, seule la durée du stade (1) du procédé est affectée. Il est donc préférable de travailler avec une quantité constante d'enzyme R 39, par exemple une quantité d'environ 3 picomoles et d'adapter selon les circonstances le volume de l'échantillon à la sensibilté souhaitée.

En pratique, on utilise des volumes d'échantillon compris entre 20 µl et 5 ml, qui permettent la détermination de très faibles concentrations en antibiotique, par exemple de l'ordre de 0,002 U.I./ml de pénicilline, en une heure ou moins.

L'excellente sensibilité, la rapidité et la précision du procédé conforme à l'invention résultent des caractéristiques particulières de l'enzyme R 39 d'une part et, d'autre part, de son immobilisation sur un support insoluble dans l'eau.

En effet, l'enzyme R 39 est caractérisée par

— la formation extrêmement rapide d'un complexe enzyme-antibiotique équimoléculaire inactif,

— la stabilité extraordinaire dudit complexe parce que, une fois formé, il ne se décompose que fort lentement. A titre d'exemple, le temps de demi-vie du complexe formé entre l'enzyme R 39 et la pénicilline G est d'environ 70 heures à 37 °C ;

— une excellente activité enzymatique se traduisant par une hydrolyse très rapide des groupements terminaux D-alanyl-D-analine du substrat peptidique.

Grâce à ces trois caractéristiques, l'enzyme R 39 occupe une position privilégiée par rapport aux D-alanyl-D-alanine-carboxypeptidases identifiées jusqu'à présent. En effet, étant donné que le temps de décomposition du complexe enzyme-antibiotique est infiniment plus grand que la durée totale qu'il faut respectivement pour la formation du complexe et pour la mesure de l'activité enzymatique résiduelle, il n'est pas à craindre que les résultats de la détermination soient faussés par une remise en liberté d'enzyme active à la suite d'une décomposition prématurée du complexe enzyme-antibiotique.

Or, lorsqu'on examine les D-alanyl-D-alanine-carboxypeptidases que l'on connaît aujourd'hui, hormis l'enzyme R 39, il n'y en a aucune autre qui répond parfaitement à ces conditions ; en effet, c'est soit la vitesse de formation du complexe qui est une dizaine de fois plus petite, soit la stabilité du complexe antibiotique-enzyme qui est absolument insuffisante, soit encore la vitesse d'hydrolyse du substrat peptidique qui est beaucoup trop faible (c'est le cas notamment de toutes les carboxypeptidases endocellulaires liées à la membrane bactérienne).

L'enzyme R 39 est la D-alanyl-D-alanine-carboxypeptidase exocellulaire soluble spécifique qui est excrétée par Actinomadura R 39, lorsqu'on cultive ce microorganisme (déposé le 10 juillet 1981 à l'Institut Pasteur à Paris n° I-127) dans un milieu de culture approprié.

Pour la mise en œuvre du procédé de l'invention, cette enzyme doit évidemment être substantiellement pure. Sa préparation et sa purification peuvent être effectuées suivant les méthodes décrites dans la littérature (voir à ce sujet l'article de J.-M. FRERE & al., Biochem. J. *143*, (1974), 233-240, intitulé « Molecular Weight, Aminoacid Composition and Physicochemical Properties of the Exocellular DD-Carboxypeptidase-Transpeptidase of Streptomyces R 39 »). Toutefois, cette enzyme, à l'état pur, est maintenant disponible dans le commerce ; on peut se la procurer auprès de la société UCB BIOPRODUCTS, S.A. (Belgique).

Selon la présente invention, l'enzyme R 39 soluble est immobilisée sur un support insoluble dans l'eau.

La méthode utilisée pour cette immobilisation n'est pas critique. On peut donc à cet effet utiliser toutes les méthodes conventionnelles connues par l'homme de métier parmi lesquelles on retiendra plus particulièrement celles qui procèdent soit par fixation de l'enzyme au support par une liaison covalente, soit par adsorption physique de l'enzyme sur le support, soit par piégeage de l'enzyme dans une matrice de polymère.

De même, la nature du matériau de support n'est pas critique. En principe, on peut utiliser n'importe quel matériau insoluble dans l'eau, organique ou inorganique, couramment employé pour l'immobilisation d'une enzyme. Comme matériau de support approprié, on peut citer des polymères naturels, tels que la cellulose et ses dérivés, l'agarose, l'amidon et ses dérivés, le dextrane, le collagène, la kératine, etc. ; des polymères synthétiques, tels que les polyacrylamides réticulés sous forme de perles ou de gels ; les polystyrènes réticulés ; les copolymères éthylène-anhydride maléique ; les polyamides ; les polyméthacrylates de 2-hydroxyéthyle réticulés ; les résines échangeuses d'ions, etc., des substances inorganiques, telles que le verre, l'alumine, la silice, le carbonate de calcium, l'hydroxyapatite, les argiles, etc.

Cependant, l'homme de métier comprendra qu'il est nécessaire de choisir une méthode d'immobilisation qui laisse intactes toutes les propriétés de l'enzyme R 39 et qui n'affecte nullement son activité enzymatique, même après une période prolongée d'entreposage.

Un matériau de support particulièrement préféré pour la mise en œuvre du procédé conforme à la présente invention est la résine de poly(N,N-diméthylacrylamide) réticulée, dont l'emploi a déjà été proposé dans la synthèse des polypeptides en phase solide (cf. R. ARSHADY et al., J. Chem. Soc. Chem. Commun. 1979, n° 9, 423-425). Cette résine présente l'avantage d'être aisément gonflable dans une grande variété de solvants tant organiques que aqueux (eau, méthanol, N,N-diméthylformamide, N,N-diméthylacétamide, pyridine ou dichlorométhane) et, en outre, d'être aisément manipulable. Elle est préparée, de préférence, par copolymérisation en émulsion d'un mélange de N,N-diméthylacrylamide, de N,N'-éthylènebisacrylamide et de l'ester méthylique de la N-acryloylsarcosine.

Dans ces conditions, on obtient une résine qui se présente sous forme de particules solides, par exemple des billes ou des perles, dont la granulométrie est de préférence comprise entre 0,1 mm et 2 mm, et qui peut donc facilement être séparée par filtration.

Cette résine possède une structure réticulée tridimensionnelle, étant donné que les chaînes du polymère sont liées entre elles par le N,N-éthylènebisacrylamide (qui agit comme agent de réticulation) et elle porte en outre des groupes esters, grâce à l'introduction de l'ester méthylique de la N-acryloylsarcosine ($CH_2=CH—CO—N(CH_3)—CH_2—COOH_3$) dans les chaînes du polymère.

Lorsque la polymérisation de cette résine est terminée, on convertit par ailleurs les groupes esters portés par cette résine en des groupes aminés fonctionnels par réaction avec de l'éthylènediamine, suivant l'équation :

$$\text{Polymère}-\underset{\overset{\|}{O}}{C}-\underset{\overset{|}{CH_3}}{N}-CH_2-COOCH_3 + H_2N-(CH_2)_2-NH_2 \longrightarrow$$

$$\text{Polymère}-\underset{\overset{\|}{O}}{C}-\underset{\overset{|}{CH_3}}{N}-CH_2-CO-HN-(CH_2)_2-NH_2$$

La résine de poly(N,N-diméthylacrylamide) ainsi modifiée contient, en général, de 0,2 à 1 mmole d'éthylènediamine par gramme de résine. Sous cette forme, elle constitue un matériau de support idéal pour l'immobilisation de l'enzyme R 39.

Cette immobilisation est réalisée de préférence par fixation de l'enzyme R 39 sur la résine par une liaison covalente. En effet, à la différence des autres méthodes d'immobilisation, cette méthode permet d'éviter les fuites de l'enzyme à partir du support.

La liaison covalente de l'enzyme R 39 sur la résine de poly(N,N-diméthylacrylamide), modifiée avec l'éthylènediamine comme on vient de l'expliquer ci-dessus, peut être réalisée par exemple au moyen d'un agent de couplage approprié contenant un groupe fonctionnel pouvant réagir avec les groupes aminés fonctionnels de la résine de poly(N,N-diméthylacrylamide) et un autre groupe fonctionnel pouvant réagir avec les groupes aminés libres de l'enzyme R 39.

On utilise de préférence les agents de couplage qui comportent des groupes esters dont le temps de demi-vie en milieu aqueux est suffisamment long, comme par exemple les diesters de N-hydroxysuccinimide d'un acide dicarboxylique aliphatique contenant 4 à 9 atomes de carbone, tel que l'acide glutarique, l'acide subérique, etc. En effet, comme la réaction de couplage avec les groupes aminés libres de l'enzyme R 39 est effectuée en milieu aqueux, il faut que les groupes fonctionnels de l'agent de couplage y soient suffisamment stables.

Pour fixer l'enzyme R 39 sur la résine de poly(N,N-diméthylacrylamide) modifiée par l'éthylènediamine, on fait réagir cette résine d'abord avec un diester de N-hydroxysuccinimide, dans un solvant organique approprié (par exemple le N,N-diméthylacétamide) pour éviter l'hydrolyse des groupes esters. Par cette réaction, les groupes aminés fonctionnels de la résine sont transformés en groupes esters fonctionnels, suivant l'équation :

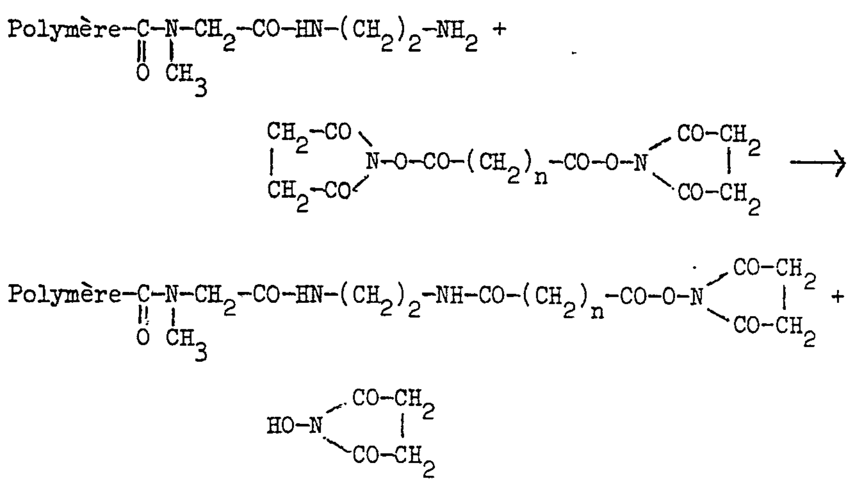

(n = un nombre entier de 2 à 7)

Ensuite, on effectue le couplage entre l'enzyme R 39 et la résine en faisant réagir les groupes esters fonctionnels, introduits sur cette résine par l'ester de N-hydroxysuccinimide, avec les groupes aminés libres de l'enzyme R 39, suivant l'équation :

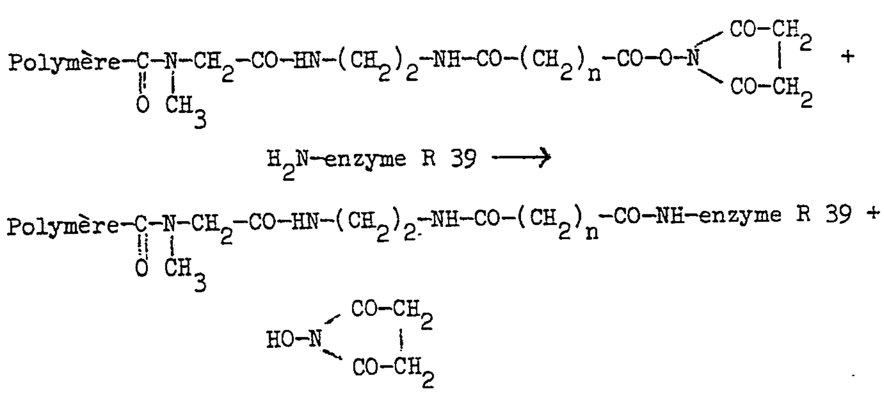

(n = un nombre entier de 2 à 7)

La réaction de couplage entre l'enzyme R 39 et la résine est effectuée pendant plusieurs heures, à une température comprise entre 0 et 20 °C et dans un tampon approprié pour ajuster le pH à une valeur comprise entre 7 et 8,5, de préférence voisine de 7,7. Il est évident que ce tampon doit être exempt d'amines primaires susceptibles de réagir avec les groupes esters fonctionnels de la résine.

Lorsque la réaction de couplage est terminée, les groupes esters n'ayant pas réagi sont bloqués par les groupes aminés de composés tels que l'éthanolamine ou le méthylester de la glycine. L'enzyme immobilisée ainsi obtenue est lavée avec un tampon approprié et elle peut être utilisée telle quelle pour la mise en œuvre du procédé conforme à la présente invention.

En utilisant cette méthode, les rendements de l'immobilisation sont excellents : ils peuvent varier entre 15 et 95 %, en fonction du pH du tampon utilisé ainsi que de la durée et de la température de la réaction de couplage.

On donne ci-après un exemple détaillé illustrant cette méthode d'immobilisation de l'enzyme R 39 sur une résine de poly(N,N-diméthylacrylamide).

Immobilisation de l'enzyme R 39 sur une résine de poly(N,N-diméthylacrylamide).

a) Préparation de la résine de poly(N,N-diméthylacrylamide) portant des groupes aminés fonctionnels dérivés de l'éthylènediamine.

La résine de poly(N,N-diméthylacrylamide) utilisée comme support a été préparée par copolymérisation en émulsion d'un mélange de N,N-diméthylacrylamide (12 g), de N,N'-éthylènebisacrylamide (1 g) et de l'ester méthylique de la N-acryloylsarcosine (1,4 g), selon la méthode décrite dans l'article de R. ARSHADY et al. (J. Chem. Soc. Chem. Comm. 1979, n° 9, 423-425).

On obtient ainsi environ 12 à 13 g de résine, sous forme de perles ayant une granulométrie comprise entre 0,1 mm et 2 mm.

On ajoute 10 g de cette résine à 320 ml d'éthylènediamine et on agite le mélange obtenu pendant une nuit à la température ambiante. Ensuite, on filtre la résine et on la lave successivement avec du N,N-diméthylformamide et de l'eau jusqu'à ce que le filtrat présente un pH neutre. Pour terminer, on lave avec du méthanol et on laisse la résine se dégonfler au moyen d'éther diéthylique.

On obtient ainsi 10 g de résine contenant 0,3 mmole d'éthylènediamine par gramme.

b) Préparation de la résine de poly(N,N-diméthylacrylamide) portant des groupes esters fonctionnels dérivés du diester de N-hydroxysuccinimide de l'acide glutarique.

b.1) Préparation du diester de N-hydroxysuccinimide de l'acide glutarique.

On dissout 6,7 g d'acide glutarique et 12,7 g de N-hydroxysuccinimide dans 50 ml de N,N-diméthylacétamide à 0 °C. On y ajoute, goutte à goutte, une solution de 23,7 g de dicyclohexylcarbodiimide dans 50 ml de dichlorométhane. On laisse revenir à la température ambiante et, après une nuit de repos, on filtre le mélange réactionnel et on évapore le filtrat. On recristallise le résidu dans de l'éther diéthylique. On obtient le diester de N-hydroxysuccinimide de l'acide glutarique avec un rendement quasi quantitatif.

b.2) Préparation de la résine de poly(N,N-diméthylacrylamide) portant des groupes esters fonctionnels.

On met 500 mg de résine de poly(N,N-diméthylacrylamide) portant des groupes aminés fonctionnels dérivés de l'éthylènediamine, préparée comme décrit en a), en suspension dans 50 ml de N,N-diméthylacétamide. On y ajoute 2 g du diester de N-hydroxysuccinimide de l'acide glutarique (préparé comme décrit en b.1). On agite le mélange obtenu pendant 24 heures à la température ambiante. On vérifie l'achèvement de la réaction par le test de E. KAISER (Anal. Biochem. *34*, (1970, n° 2), 595-8). On filtre la résine et on la lave cinq fois avec 100 ml de N,N-diméthylacétamide et cinq fois avec 100 ml de méthanol, puis on laisse la résine se dégonfler au moyen d'éther diéthylique.

c) Immobilisation de l'enzyme R 39 sur la résine de poly(N,N-diméthylacrylamide) portant des groupes esters fonctionnels dérivés du diester de N-hydroxysuccinimide de l'acide glutarique.

c.1) Préparation de l'enzyme R 39.

L'enzyme R 39 utilisée a été préparée et purifiée suivant la méthode décrite dans l'article de J.-M. FRERE et al. (Biochem. J. *143*, (1974), 233-240).

L'enzyme R 39 ainsi purifiée possède une activité spécifique de 19,8 unités/mg de protéine. Une unité d'enzyme R 39 catalyse l'hydrolyse de 1 µmole de $N^{alpha}$, $N^{epsilon}$-diacétyl-L-lysyl-D-alanyl-D-alanine par minute à 37 °C, lorsque l'enzyme est incubée avec une solution 8 mM du substrat dans un tampon Tris-HCl 0,03 M (pH 7,5) contenant du $MgCl_2$ 3mM.

On soumet 600 µg d'enzyme R 39 purifiée, dissoute dans 1 ml de tampon Tris-HCl 0,1 M (pH 7,7) contenant du NaCl 0,2 M et du $MgCl_2$ 0,05 M, à une dialyse pendant 6 heures contre 200 ml de tampon Hepes 0,1 M (pH 7,7) contenant du NaCl 0,1 M et du $MgCl_2$ 0,05 M. On répète cette opération quatre fois.

Tris-HCl = chlorhydrate de 2-amino-2-hydroxyméthyl-1,3-propanediol ;

Hepes = acide 4-hydroxyéthyl-1-pipérazineéthanesulfonique.

c.2) Préparation de la résine.

On laisse 100 mg de résine de poly(N,N-diméthylacrylamide) portant des groupes esters fonctionnels dérivés du diester de N-hydroxysuccinimide de l'acide glutarique, préparée comme décrit en b.2), se gonfler dans 50 ml de N,N-diméthylacétamide. Lorsque les perles ont atteint leur point de gonflement maximum (après environ trois heures), on filtre la résine et on la lave cinq fois avec 100 ml de tampon Hepes 0,1 M (pH 7,7) et cinq fois avec 100 ml de tampon Hepes 0,1 M (pH 7,7) contenant du NaCl 0,1 M et du $MgCl_2$ 0,05 M.

c.3) Couplage entre l'enzyme R 39 et la résine.

On place la résine humide, préparée comme décrit en c.2), dans une fiole de 10 ml. On a ajouté 70 µg (1 320 picomoles) d'enzyme R 39, préparée comme décrit en c.1), dissoute dans 1,75 ml de tampon Hepes 0,1 M (pH 7,7) contenant du NaCl 0,1 M et du $MgCl_2$ 0,05 M. Puis, on soumet la fiole à un mouvement de rotation de 100 tours par minute pendant 16 heures à la température ambiante.

On filtre la résine et la lave cinq fois avec 10 ml de tampon Hepes 0,1 M (pH 7,7) contenant du NaCl 0,1 M et du $MgCl_2$ 0,05 M.

Après cette opération, on soumet la résine au vide de la pompe à eau pendant 30 minutes.

Le poids de la résine ainsi obtenue est de 503 mg. L'activité enzymatique de l'enzyme R 39 immobilisée sur cette résine correspond à 15 µg (283 picomoles). Le rendement de l'immobilisation est donc de 21 %.

L'enzyme R 39 immobilisée sur un support solide possède une excellente stabilité et elle supporte par conséquent des températures élevées pouvant aller jusque 70 °C. C'est pourquoi, l'incubation du liquide biologique avec l'enzyme R 39 immobilisée peut être effectuée dans un intervalle de température compris entre 20 et 50 °C. De préférence, cette température est voisine de 37 °C. En effet, dans ces conditions, la durée de l'incubation, qui est étroitement liée au temps nécessaire à la formation du complexe enzyme-antibiotique équimoléculaire inactif, est d'environ 20 minutes. Une augmentation de la température de l'incubation aura pour effet une diminution de sa durée et inversement. Il est donc possible de raccourcir encore davantage la durée du procédé en augmentant la température.

Conformément à la présente invention, au terme du stade (1) du procédé, l'enzyme R 39 immobilisée est séparée du liquide biologique. Cette séparation peut être exécutée par toute méthode appropriée, par exemple par décantation, par filtration ou encore par centrifugation. Une simple filtration est préférée. On lave ensuite l'enzyme immobilisée, une ou plusieurs fois, avec un tampon approprié afin d'éliminer les traces de liquide biologique qui pourraient y subsister.

Au stade (2) du procédé conforme à la présente invention, l'enzyme immobilisée (débarrassée du liquide biologique) est incubée avec une quantité déterminée de substrat en solution.

Au cours de ce stade, la fraction de l'enzyme qui n'a pas été consommée dans la formation du complexe enzyme-antibiotique au stade (1) du procédé, est utilisée pour effectuer une hydrolyse d'un substrat spécifique de l'enzyme R 39. Cette réaction d'hydrolyse va donc produire une quantité de D-alanine correspondant à l'activité résiduelle de l'enzyme R 39.

Etant donné la nature même de l'enzyme R 39, il est évident que l'on pourra utiliser, en tant que substrat, tous les peptides qui possèdent des groupements terminaux D-alanyl-D-alanine. Uniquement à titre d'exemples non limitatifs, citons la $N^{alpha}$, $N^{epsilon}$-diacétyl-L-lysyl-D-alanyl-D-alanine, la $N^{alpha}$-acétyl-L-lysyl-D-alanyl-D-alanine, etc.

La quantité de substrat mise en œuvre n'est pas critique pour autant que l'on utilise une quantité de substrat largement en excès par rapport à la quantité d'enzyme R 39 mise en œuvre. En effet, il faut toujours opérer dans des conditions de saturation de l'enzyme par le substrat.

Les conditions opératoires à observer au cours de ce stade sont sensiblement les mêmes que celles indiquées plus haut pour le stade (1). L'incubation peut être effectuée dans un intervalle de température compris entre 20 et 50 °C, de préférence à une température voisine de 37 °C. La durée de l'incubation doit être au moins suffisante pour obtenir une quantité mesurable de D-alanine avec l'enzyme R 39 non inactivée. Cette durée est de préférence d'environ 30 minutes pour une température d'incubation de 37 °C. Cette durée peut être diminuée en augmentant la température de l'incubation ou, au contraire, augmentée en diminuant la température de l'incubation. On a donc également intérêt à augmenter la température de cette incubation dans tous les cas où la rapidité de la détermination est un facteur important.

Afin de conserver une activité enzymatique optimale, le milieu d'incubation devra, de préférence, présenter une valeur de pH comprise entre 7 et 8,5. Habituellement on maintient une valeur de pH voisine de 7,7 en effectuant l'incubation au sein d'un tampon approprié.

Au stade (3) du procédé conforme à la présente invention, on détermine la quantité de D-alanine formée au stade (2).

Cette détermination de la D-alanine peut être effectuée par n'importe quelle méthode connue en soi. Il importe seulement qu'elle soit rapide, peu onéreuse et qu'elle permette de déterminer spécifiquement la D-alanine à l'exclusion de tous les autres produits qui sont présents dans le liquide à examiner.

C'est pourquoi les méthodes enzymatiques permettant un dosage colorimétrique de la D-alanine sont celles que l'on préfère.

Une méthode particulièrement préférée est la méthode enzymatique décrite par J.-M. FRERE et al. dans « Methods in Enzymology, Vol. XLV, partie B, (1976), 610-636 » ; plus particulièrement aux pages 612 et 613.

Dans cette méthode, la D-alanine est oxydée en acide pyruvique à l'aide d'une D-aminoacide-oxydase (ensemble avec son coenzyme, le flavine-adénine-dinucléotide) ; il se forme en même temps une quantité de peroxyde d'hydrogène correspondant à la quantité de D-alanine. Ce peroxyde d'hydrogène est à son tour utilisé pour oxyder l'o-dianisidine à l'aide d'une peroxydase. Comme la quantité d'o-dianisidine oxydée ainsi formée dépend étroitement de la quantité de D-alanine et que l'o-dianisidine oxydée produit une coloration, la mesure de l'intensité de la couleur ainsi produite peut être mise à profit pour la détermination de la quantité de D-alanine par une méthode de dosage colorimétrique.

Il est évident que ce système est basé essentiellement sur l'utilisation d'une substance, en l'occurrence l'o-dianisidine, dont la forme oxydée et la forme réduite présentent des couleurs différentes. De telles substances sont connues dans la technique sous le nom d'« indicateurs redox ».

Il existe actuellement un grand nombre d'indicateurs redox qui peuvent remplacer utilement l'o-dianisidine dans la méthode précitée. Citons à titre d'exemple le sel d'ammonium de l'acide 2,2'-azinobis-(3-éthyl-2,3-dihydro-6-benzothiazolesulfonique), la 4-aminoantipyrine en présence de phénol ou de N,N-diméthylaniline, etc.

C'est pourquoi, selon une forme de réalisation préférée, la quantité de D-alanine est déterminée par incubation du mélange obtenu au stade (2) avec d'une part une D-aminoacide-oxydase, qui catalyse l'oxydation de la D-alanine en acide pyruvique (avec formation simultanée de peroxyde d'hydrogène) et avec d'autre part une peroxydase et un indicateur redox, lequel est oxydé par le peroxyde d'hydrogène formé, au moyen de la peroxydase, pour produire une coloration dont l'intensité est fonction de la quantité de D-alanine.

Conjointement avec la D-aminoacide-oxydase, on utilise toujours son coenzyme, le flavine-adénine-dinucléotide (FAD).

Dans cette forme de réalisation préférée, on peut en principe employer tout indicateur redox capable d'être oxydé par le peroxyde d'hydrogène, en présence de peroxydase comme catalyseur. Des indicateurs redox particulièrement préférés sont l'o-dianisidine, dont la forme oxydée présente une coloration brune (maximum d'absorption à $\lambda = 460$ nm) et le sel d'ammonium de l'acide 2,2'-azinobis(3-éthyl-2,3-dihydro-6-benzothiazolesulfonique), dont la forme oxydée présente une coloration verte (maximum d'absorption à $\lambda = 420$ nm).

On notera que la coloration brune de l'o-dianisidine oxydée peut être changée avantageusement en une coloration rose vif par l'addition d'acide sulfurique. Cette coloration rose est très stable, ce qui est très important lorsqu'on désire maintenir le résultat de la détermination pendant un certain temps.

En outre, dans le but d'assurer une détermination rapide, il est possible d'exécuter les stades (2) et (3) simultanément. Dans cette forme de réalisation, on ajoute directement au stade (2) du procédé les réactifs permettant la détermination de la D-alanine au mélange de l'enzyme immobilisée et de la solution de substrat et on effectue une seule incubation de l'ensemble, dans des conditons sensiblement identiques à celles indiquées plus haut pour le stade (2).

Au stade (4) du procédé, la détermination du stade (3) est comparée avec un étalon pour obtenir la concentration de l'antibiotique dans le liquide biologique.

La détermination quantitative de la concentration en antibiotique peut être effectuée selon la méthode qui suit.

Tout d'abord, on prépare une série d'échantillons du liquide biologique renfermant une concentration connue en antibiotique bêta-lactame.

Cette série contiendra, en plus d'un certain nombre d'échantillons contenant une concentration croissante en antibiotique, deux échantillons du liquide biologique exempts d'antibiotique.

Ensuite, on traite tous ces échantillons de manière rigoureusement identique en suivant les stades (1), (2) et (3) du procédé conforme à la présente invention.

Pour l'un des échantillons exempt d'antibiotique, on remplace toutefois la solution du substrat utilisée au stade (2) par un volume identique d'eau. Dans ce cas particulier, on obtiendra donc, au terme du stade (3), une solution qui contient tous les réactifs à l'exception du substrat. Etant donné l'absence dudit substrat, il ne se formera donc pas de D-alanine et par voie de conséquence l'o-dianisidine ne sera pas oxydée. La solution obtenue présentera une coloration jaune clair. Cet échantillon sera désigné ci-après par « échantillon blanc ».

L'autre échantillon exempt d'antibiotique produit au contraire une coloration brune prononcée. En effet, comme l'échantillon est exempt d'antibiotique, l'enzyme R 39 n'est pas inactivée au stade (1) et il se formera une quantité maximum de D-alanine (correspondant à l'activité totale de l'enzyme R 39 mise en œuvre) et donc une quantité maximum d'o-dianisidine oxydée et par voie de conséquence il se produira une coloration brune prononcée. Cet échantillon sera désigné ci-après par « échantillon témoin ».

On comprendra aussi que lorsque les échantillons contiennent une quantité molaire d'antibiotique qui est inférieure à la quantité molaire d'enzyme R 39 mise en œuvre, une fraction seulement de cette enzyme sera inactivée par l'antibiotique au stade (1) ; dans ces cas, il se formera donc une quantité de D-alanine qui correspondra à l'activité résiduelle de la fraction de l'enzyme qui n'a pas été inactivée par l'antibiotique et par conséquent aussi une quantité correspondante d'o-dianisidine oxydée. Dans ces cas, il se produira donc également une coloration brune, mais d'une intensité inférieure à celle observée avec l'échantillon témoin.

Enfin, lorsque les échantillons contiennent une quantité molaire d'antibiotique qui est égale ou supérieure à la quantité molaire d'enzyme R 39 mise en œuvre, l'enzyme sera complètement inactivée par l'antibiotique au cours du stade (1) ; il ne se formera donc pas de D-alanine et l'o-dianisidine ne sera pas oxydée. Dans ces cas, on obtiendra donc une coloration jaune clair identique à celle observée avec l'échantillon blanc.

Pour obtenir une détermination précise, on mesure au spectrophotomètre la densité optique des colorations obtenues respectivement avec tous les échantillons y compris les échantillons blanc et témoin.

Comme on trouve aussi pour l'échantillon blanc une certaine valeur de densité optique, il est nécessaire de déduire cette valeur de celles trouvées respectivement pour l'échantillon témoin et pour les autres échantillons.

A partir des valeurs de densité optique ainsi obtenues, on calcule le pourcentage d'activité résiduelle (de l'enzyme R 39) pour chaque échantillon. Ce pourcentage est égal au rapport, multiplié par 100, entre les valeurs de densité optique trouvées respectivement pour un échantillon déterminé et pour l'échantillon témoin.

On établit ensuite un graphique, portant en abscisses, les concentrations en antibiotique et, en ordonnées, les pourcentages d'activité résiduelle de l'enzyme R 39.

On obtient une droite qui coupe respectivement l'ordonnée en un point correspondant à l'échantillon témoin (100 % d'activité enzymatique résiduelle) et l'abscisse en un point correspondant à l'échantillon contenant une quantité d'antibiotique égale à la quantité molaire d'enzyme R 39 mise en œuvre (0 % d'activité enzymatique résiduelle).

Le graphique ainsi obtenu constitue un « étalon » qui permet de déterminer une concentration inconnue en antibiotique bêta-lactame dans un échantillon du liquide biologique ayant servi à son établissement. A cet effet, on traite cet échantillon de manière rigoureusement identique en suivant les stades (1), (2) et (3) du procédé conforme à l'invention ; on mesure au spectrophotomètre la densité optique de la coloration obtenue ; on en retranche la valeur de la densité optique trouvée pour l'échantillon blanc ; on calcule le pourcentage d'activité enzymatique résiduelle de la manière indiquée plus haut et on obtient la concentration en antibiotique de l'échantillon en se reportant à l'étalon.

On peut ainsi déterminer de manière quantitative des concentrations en antibiotique aussi faibles que 0,000 5 U.I./ml de liquide biologique en un peu moins d'une heure.

Cependant cette méthode nécessite l'emploi d'un spectrophotomètre et doit donc, en principe, être exécutée dans un laboratoire. Or, si l'on envisage uniquement de déterminer si la concentration en antibiotique dépasse ou non un certain seuil (par exemple une concentration maximum imposée par l'industrie laitière), il n'est pas nécessaire d'avoir recours à un spectrophotomètre. Ceci nécessite cependant au préalable quelques explications.

Comme on l'a vu plus haut, la courbe d'étalonnage coupe l'abscisse en un point correspondant à un échantillon contenant une quantité d'antibiotique égale à la quantité molaire d'enzyme R 39 mise en œuvre. A cette concentration critique, le pourcentage d'activité enzymatique résiduelle est de zéro %, parce que au terme du stade (3) du procédé conforme à l'invention, on obtient une coloration jaune clair identique à celle obtenue avec l'échantillon blanc.

Au-dessus de cette concentration critique, on obtient également une coloration jaune clair, parce que le pourcentage d'activité enzymatique résiduelle est toujours de zéro %. Par contre, au-dessous de cette concentration critique, on obtient une coloration brune parce qu'il subsiste un certain pourcentage d'activité enzymatique résiduelle.

Par conséquent, en se basant simplement sur la coloration observée au terme du stade (3) du procédé, on peut évaluer directement dans un échantillon si la concentration en antibiotique dépasse ou non ladite concentration critique.

Il suffit donc de connaître ladite concentration critique, pour que l'on puisse ensuite, sans avoir recours à un spectrophotomètre, déterminer rapidement si un échantillon contient une concentration en antibiotique bêta-lactame qui dépasse (ou non) cette concentration critique. A cet effet, on traite cet échantillon de manière identique suivant les stades (1), (2) et (3) du procédé conforme à l'invention et ensuite on observe simplement la coloration obtenue ; si celle-ci est jaune clair, la concentration en antibiotique sera au moins égale à la concentration critique ; si par contre, elle est brune, la concentration en antibiotique sera inférieure à la concentration critique.

On peut ainsi déterminer, visuellement et avec certitude, si des échantillons renferment plus ou moins de 0,002 U.I. d'antibiotique par ml de liquide biologique et ce en un temps inférieur à une heure.

Cette méthode convient donc parfaitement pour l'examen en série d'échantillons de lait, même en dehors d'un laboratoire, par exemple sur place à la ferme, par du personnel non spécialisé.

La méthode de détermination quantitative et qualitative de la concentration en antibiotique conforme à la présente invention, vient d'être expliquée en détail en se référant plus particulièrement au changement de couleur produit par l'o-dianisidine. Cependant, l'homme de métier comprendra aisément

qu'en se servant dans cette méthode d'autres indicateurs redox, seule la couleur observée sera différente.

Un autre objet de la présente invention est de procurer une trousse d'essai utilisable pour la mise en œuvre du procédé conforme à l'invention, c'est-à-dire pouvant servir à la détermination d'antibiotiques à noyau bêta-lactame dans un liquide biologique.

Cette trousse comprend notamment

1) une quantité déterminée de D-alanyl-D-alanine-carboxypeptidase soluble produite par Actinomadura R 39, ladite enzyme étant immobilisée sur un support insoluble dans l'eau ;

2) une quantité déterminée de substrat ;

3) les réactifs permettant la détermination de la D-alanine ; et

4) éventuellement un étalon, par rapport auquel les résultats d'essais réalisés avec les réactifs (1), (2) et (3) peuvent être comparés.

Selon une forme de réalisation préférée, l'enzyme R 39 est immobilisée sur une résine de poly(N,N-diméthylacrylamide) réticulée, le substrat est le tripeptide $N^{alpha}$, $N^{epsilon}$-diacétyl-L-lysyl-D-alanyl-D-alanine, tandis que les réactifs sont constitués par (a) une D-aminoacide-oxydase (conjointement avec son coenzyme, le flavine-adénine-dinucléotide), (b) une peroxydase et (c) un indicateur redox, par exemple l'o-dianisidine.

En tant qu'étalon, on peut éventuellement incorporer dans la trousse un graphique établissant la relation entre les concentrations en antibiotique et les pourcentages d'activité résiduelle de l'enzyme R 39. On peut ainsi réaliser des déterminations quantitatives ainsi qu'il a été expliqué plus haut. Ce graphique n'est toutefois pas indispensable. En effet, si la trousse doit servir uniquement pour déterminer si la concentration en antibiotique dépasse ou non une certaine valeur limite, il suffit d'indiquer dans le mode d'emploi à quelles concentrations en antibiotique on observe un virage de la coloration, après traitement de l'échantillon selon le procédé conforme à l'invention.

Selon une forme de réalisation particulièrement avantageuse, l'enzyme immobilisée est placée dans une seringue munie dans son fond d'une plaque de matière filtrante, par exemple du verre fritté. En effet, ce dispositif facilite la séparation par filtration de l'enzyme R 39 immobilisée à partir du liquide biologique, de même que son lavage, au terme du stade (1) du procédé conforme à l'invention.

Les exemples suivants illustrent la présente invention, sans toutefois la limilter.

## Exemple 1

Cet exemple illustre l'application du procédé conforme à la présente invention pour la détermination de faibles concentrations de pénicilline G dans le lait.

(a) Préparation des réactifs.

1) Enzyme R 39 immobilisée.

L'enzyme R 39 a été immobilisée sur une résine de poly(N,N-diméthylacrylamide) suivant la méthode décrite dans l'exemple donné précédemment dans la description.

2) Solution de substrat.

On prépare une solution contenant 6 mg de $N^{alpha}$, $N^{epsilon}$-diacétyl-L-lysyl-D-alanyl-D-alanine dans 1 ml d'eau.

3) Suspension de D-aminoacide-oxydase.

On prépare une suspension contenant 5 mg de D-aminoacide-oxydase (activité spécifique : 15 U/mg) par ml d'une solution aqueuse 3 molaire de sulfate d'ammonium.

4) Solution de flavine-adénine-dinucléotide (FAD).

On prépare une solution contenant 500 µg de FAD dans 6 ml de tampon Tris-HCl 0,1 M (pH : 8,0);

5) Solution de peroxydase.

On prépare une solution contenant 50 µg de peroxydase par ml d'eau.

6) Solution d'o-dianisidine.

On prépare une solution contenant 2,6 mg de dichlorhydrate d'o-dianisidine par 500 µl d'eau.

b) Mode opératoire.

On prépare une série d'échantillons de 1 ml de lait contenant chacun une concentration connue en pénicilline G, ainsi que deux échantillons (blanc et témoin) de 1 ml de lait, exempts de pénicilline G.

A chaque échantillon, on ajoute 5,5 mg d'enzyme R 39 immobilisée (= 3 picomoles d'enzyme) et on incube le mélange pendant 20 minutes à 37 °C.

On sépare ensuite, par filtration, le lait de l'enzyme immobilisée et on lave l'enzyme immobilisée trois fois avec 1 ml de tampon Hepes 0,1 M (pH = 7,7) contenant du NaCl 0,1 M et du $MgCl_2$ 0,05 M.

Puis on ajoute à l'enzyme immobilisée ainsi séparée et lavée, soit 20 µl de tampon Hepes 0,1 M (pH = 7,7) contenant du NaCl 0,1 M et du $MgCl_2$ 0,05 M et 10 µl de la solution de substrat (dans le cas de l'échantillon témoin et des échantillons contenant de la pénicilline G), soit 20 µl du tampon Hepes et 10 µl d'eau (dans le cas de l'échantillon blanc). On incube le mélange pendant 30 minutes à 37 °C.

Au terme de cette incubation, on ajoute au produit obtenu respectivement 2 µl de la suspension de

D-aminoacide-oxydase, 60 µl de la solution de FAD, 10 µl de la solution de peroxydase et 5 µl de la solution d'o-dianisidine ; ensuite on incube le mélange une nouvelle fois pendant 10 minutes à 37 °C.

On mesure ensuite au spectrophotomètre la densité optique des solutions colorées obtenues. A cet effet, on ajoute 1 ml d'une solution méthanol-eau (proportions 1 : 1) dans la cuvette et on mesure la densité optique à 460 nm.

On déduit la valeur de densité optique trouvée pour l'échantillon blanc de celles trouvées respectivement pour l'échantillon témoin et pour les autres échantillons. Enfin, on calcule le pourcentage d'activité enzymatique résiduelle pour chaque échantillon à partir des valeurs de densité optique ainsi obtenues.

Les résultats obtenus avec deux séries d'échantillons de 1 ml de lait renfermant des concentrations variables en pénicilline G sont reproduits au tableau I

Tableau I

1re série

| A | B | C | Δ | % | D |
|---|---|---|---|---|---|
| 1 | 0,008 2 | 0,035 | 0,000 | 0 | jaune clair |
| 2 | 0,004 1 | 0,040 | 0,005 | 3 | jaune clair |
| 3 | 0,002 0 | 0,035 | 0,000 | 0 | jaune clair |
| 4 | 0,001 0 | 0,150 | 0,115 | 69 | jaune brun |
| 5 | 0,000 5 | 0,170 | 0,135 | 82 | brune |
| témoin | 0 | 0,200 | 0,165 | 100 | brune |
| blanc | 0 | 0,035 | — | — | jaune clair |

2e série

| A | B | C | Δ | % | D |
|---|---|---|---|---|---|
| 1 | 0,002 0 | 0,040 | 0,005 | 3 | jaune clair |
| 2 | 0,001 5 | 0,055 | 0,020 | 12 | jaune |
| 3 | 0,001 0 | 0,100 | 0,065 | 41 | jaune brun |
| 4 | 0,000 5 | 0,145 | 0,110 | 69 | jaune brun |
| témoin | 0 | 0,195 | 0,160 | 100 | brune |
| blanc | 0 | 0,035 | — | — | jaune clair |

A = numéro de l'échantillon ;
B = concentration en pénicilline G (U.I./ml) ;
C = densité optique à 460 nm ;
Δ = densité optique trouvée (colonne 3) — densité optique de l'échantillon blanc ;
% = pourcentage d'activité enzymatique résiduelle ;
D = coloration observée.

Les résultats ainsi obtenus sont reproduits sous forme de graphique, représenté sur le dessin d'accompagnement. Sur ce graphique, on a porté, en abscisses, les concentrations en pénicilline G en U.I./ml et, en ordonnées, les pourcentages (symbole %) d'activité enzymatique résiduelle.

Ce graphique constitue un étalon qui permet de déterminer la concentration en pénicilline G dans d'autres échantillons de 1 ml de lait. A cet effet, on traite ces échantillons de la manière décrite dans cet exemple et on détermine leur concentration respective en pénicilline G en se reportant à l'étalon.

Il ressort du tableau I que pour un échantillon contenant 0,002 U.I. de pénicilline ou plus par ml de lait, on trouve une activité enzymatique résiduelle de l'ordre de zéro % et on observe une coloration jaune clair identique à celle de l'échantillon blanc. D'autre part, pour les échantillons renfermant une quantité inférieure à 0,002 U.I. de pénicilline par ml de lait, on trouve un certain pourcentage d'activité enzymatique résiduelle et on observe une coloration jaune brun ou brune.

Par conséquent, en se basant simplement sur la coloration observée au terme de la dernière incubation, on peut déterminer, visuellement et avec certitude, si un échantillon de lait contient une concentration au moins égale à 0,002 U.I. de pénicilline par ml de lait soit 1,2 ng/ml de lait. En effet, à cette concentration ou à une concentration plus élevée, on obtient une coloration jaune clair identique à celle de l'échantillon blanc. Par contre, au-dessous de cette concentration (par exemple si l'échantillon est exempt de pénicilline), la coloration vire du jaune clair au brun.

On voit donc que le procédé conforme à l'invention permet d'effectuer très rapidement une détermination de très faibles concentrations de pénicilline dans le lait sans devoir recourir à un spectrophotomètre ou à un autre appareil similaire d'analyse. Ceci montre l'intérêt de ce procédé, qui permet d'effectuer des déterminations rapides et en série dans le lait sur le lieu même de sa collecte, c'est-à-dire à la ferme.

En outre, il est évident que ce procédé peut également être utilisé pour effectuer des déterminations

quantitatives au laboratoire. En effet, le graphique représenté sur le dessin d'accompagnement montre clairement qu'il est possible, en utilisant un spectrophotomètre, de déterminer des concentrations aussi faibles que 0,000 5 U.I. de pénicilline par ml de lait, soit 0,3 ng/ml de lait.

### Exemple 2

Cet exemple illustre l'application du procédé conforme à la présente invention, pour la détermination de faibles concentrations de pénicilline G dans le sérum et dans l'urine.

On procède comme à l'exemple 1 et en utilisant les mêmes réactifs, mais on remplace les échantillons de 1 ml de lait par des échantillons de 1 ml de sérum ou de 1 ml d'urine.

Les résultats obtenus sont reproduits aux tableaux II et III, respectivement pour le sérum et l'urine.

Tableau II (sérum)

| A | B | C | Δ | % | D |
|---|---|---|---|---|---|
| 1 | 0,010 | 0,030 | 0,000 | 0 | jaune clair |
| 2 | 0,003 | 0,035 | 0,005 | 4 | jaune clair |
| témoin | 0 | 0,145 | 0,115 | 100 | brune |
| blanc | 0 | 0,030 | — | — | jaune clair |

Tableau III (urine)

| A | B | C | Δ | % | D |
|---|---|---|---|---|---|
| 1 | 0,010 | 0,035 | 0,005 | 4 | jaune clair |
| 2 | 0,003 | 0,040 | 0,010 | 8 | jaune clair |
| témoin | 0 | 0,150 | 0,120 | 100 | brune |
| blanc | 0 | 0,030 | — | — | jaune clair |

Les tableaux II et III montrent clairement que le procédé conforme à l'invention peut être appliqué avec succès pour déterminer visuellement si un échantillon de sérum ou d'urine contient une concentration au moins égale à 0,003 U.I. de pénicilline par ml de sérum ou d'urine.

En effet, à cette concentration ou à une concentration plus élevée, la coloration observée sera jaune clair.

Il est à remarquer que, dans le cas de l'urine, il est impossible d'effectuer une telle détermination par la méthode de J.-M. FRERE, dans laquelle on utilise l'enzyme R 39 soluble mais non immobilisée et ce, même sur des volumes d'urine aussi faibles que 10 μl.

### Exemple 3

Cet exemple illustre l'application du procédé conforme à la présente invention au dosage de la céphalosporine C dans le sérum.

On procède comme à l'exemple 1 et en utilisant les mêmes réactifs, mais on remplace les échantillons de 1 ml de lait par des échantillons de 1 ml de sérum contenant chacun une concentration connue en céphalosporine C.

Les résultats obtenus sont reproduits dans le tableau IV suivant :

Tableau IV

| A | B* | C | Δ | % | D |
|---|---|---|---|---|---|
| 1 | 10 | 0,030 | 0,000 | 0 | jaune clair |
| 2 | 2 | 0,040 | 0,010 | 8 | jaune clair |
| 3 | 1 | 0,085 | 0,055 | 44 | jaune brun |
| témoin | 0 | 0,155 | 0,125 | 100 | brune |
| blanc | 0 | 0,030 | — | — | jaune clair |

*B = concentration en céphalosporine C (ng/ml).

Exemple 4

Cet exemple illustre l'application du procédé conforme à la présente invention au dosage de la céphalosporine C dans le sérum et exécuté à une température plus élevée.

On procède comme à l'exemple 3, mais pour chaque incubation, on opère à la température de 47 °C et on réduit de moitié la durée de chaque incubation.

En outre, à chaque échantillon de 1 ml de sérum, on ajoute au préalable 100 µl de tampon Hepes 0,5 M (pH 7,7) contenant du NaCl 0,5 M et du $MgCl_2$ 0,25 M.

Les résultats obtenus sont reproduits au tableau V suivant :

Tableau V

| A | B* | C | Δ | % | D |
|---|---|---|---|---|---|
| 1 | 10 | 0,040 | 0,005 | 0 | jaune clair |
| 2 | 2 | 0,035 | 0,000 | 0 | jaune clair |
| 3 | 1 | 0,095 | 0,060 | 41 | jaune brun |
| témoin | 0 | 0,180 | 0,145 | 100 | brune |
| blanc | 0 | 0,035 | — | — | jaune clair |

*B = concentration en céphalosporine C (ng/ml).

Cet exemple montre clairement qu'en augmentant la température d'incubation il est possible de diminuer de façon sensible la durée du procédé.

**Revendications**

1. Procédé enzymatique pour la détermination d'antibiotiques à noyau bêta-lactame dans un liquide biologique comprenant les stades suivants :

(1) l'incubation dudit liquide avec la D-alanyl-D-alanine-carboxypeptidase soluble produite par Actinomadura R 39 portant le numéro de souche I-127 à la C.N.C.M., cette incubation étant menée dans des conditions permettant à l'antibiotique bêta-lactame éventuellement présent dans ledit liquide de réagir avec l'enzyme pour former un complexe enzyme-antibiotique équimoléculaire, inactif et sensiblement irréversible ;

(2) l'incubation du produit obtenu à la fin du stade (1) avec une solution de substrat dans des conditions permettant l'hydrolyse dudit substrat par l'enzyme pour former une quantité de D-alanine correspondant à l'activité enzymatique résiduelle ;

(3) la détermination de la quantité de D-alanine formée au stade (2) ;

(4) la comparaison de la détermination du stade (3) avec un étalon pour obtenir la concentration de l'antibiotique dans le liquide biologique,

caractérisé en ce que l'enzyme mise en œuvre est immobilisée sur un support insoluble dans l'eau et en ce que cette enzyme immobilisée est séparée à partir du liquide biologique et lavée, avant d'être incubée avec la solution de substrat au stade (2).

2. Procédé selon la revendication 1, caractérisé en ce que le support insoluble dans l'eau est une résine de poly(N,N-diméthylacrylamide) réticulée.

3. Procédé selon la revendication 2, caractérisé en ce que l'immobilisation est réalisée par fixation de l'enzyme sur la résine de poly(N,N-diméthylacrylamide) par une liaison covalente.

4. Procédé selon l'une quelconque des revendications 1 à 3, caractérisé en ce que les stades (2) et (3) sont exécutés simultanément.

5. Procédé selon l'une quelconque des revendications 1 à 4, caractérisé en ce que le liquide biologique est choisi parmi le lait, le sérum, l'urine, le sang, la salive, les extraits de viande et les liquides de fermentation.

6. Procédé selon l'une quelconque des revendications 1 à 5, caractérisé en ce que l'antibiotique à déterminer est choisi parmi la benzylpénicilline, l'ampicilline, la phénoxyméthylpénicilline, la carbénicilline, la méthicilline, l'oxacilline, la cloxacilline, la céphalosporine C, la céfaloglycine, la céfalothine et la céfalexine.

7. Procédé selon l'une quelconque des revendications 1 à 6, caractérisé en ce que le substrat est choisi parmi la $N^{alpha}$, $N^{epsilon}$-diacétyl-L-lysyl-D-alanyl-D-alanine ou la $N^{alpha}$-acétyl-L-lysyl-D-alanyl-D-alanine.

8. Procédé selon l'une quelconque des revendications 1 à 7, caractérisé en ce que la quantité de D-alanine est déterminée par incubation du mélange obtenu au stade (2) avec, d'une part, une D-aminoacide-oxydase, qui catalyse l'oxydation de la D-alanine en acide pyruvique, avec formation simultanée de peroxyde d'hydrogène et, d'autre part, une peroxydase et un indicateur redox, lequel est oxydé par le peroxyde d'hydrogène formé, au moyen de la peroxydase, pour produire une coloration dont l'intensité est fonction de la quantité de D-alanine.

9. Trousse d'essai pour la détermination d'antibiotiques à noyau bêta-lactame dans un liquide biologique comprenant :

(1) une quantité déterminée de D-alanyl-D-alanine-carboxypeptidase soluble produite par Actinomadura R 39, portant le numéro de souche I-127 à la C.N.C.M., ladite enzyme étant immobilisée sur un support insoluble dans l'eau ;

(2) une quantité déterminée de substrat ;

(3) les réactifs permettant la détermination de la D-alanine ; et

(4) éventuellement un étalon, par rapport auquel les résultats d'essais réalisés avec les réactifs (1), (2) et (3) peuvent être comparés.

10. Trousse d'essai selon la revendication 9, caractérisé en ce que le support insoluble dans l'eau est une résine de poly(N,N-diméthylacrylamide) réticulée.

11. Trousse d'essai selon l'une quelconque des revendications 9 et 10, caractérisée en ce que le substrat est choisi parmi la $N^{alpha}$, $N^{epsilon}$-diacétyl-L-lysyl-D-alanyl-D-alanine ou la $N^{alpha}$-acétyl-L-lysyl-D-alanyl-D-alanine.

12. Trousse d'essai selon l'une quelconque des revendications 9 à 11, caractérisée en ce que les réactifs (3) comprennent a) une D-aminoacide-oxydase, b) une peroxydase et c) un indicateur redox.

**Claims**

1. Enzymatic process for the determination of antibiotics containing a beta-lactam nucleus in a biological liquid comprising the following steps :

(1) incubation of the said liquid with the soluble D-alanyl-D-alanine-carboxypeptidase produced by Actinomadura R 39 bearing the strain number I-127 at the C.N.C.M., this incubation being conducted under conditions allowing the beta-lactam antibiotic which may be present in the said liquid to react with the enzyme to form an inactive and substantially irreversible equimolecular enzyme-antibiotic complex ;

(2) incubation of the product obtained at the end of step (1) with a substrate solution under conditions allowing the said substrate to be hydrolysed by the enzyme to form an amount of D-alanine corresponding to the residual enzymatic activity ;

(3) determination of the amount of D-alanine formed in step (2) ;

(4) comparison of the determination of step (3) with a standard in order to obtain the concentration of the antibiotic in the biological liquid,

characterised in that the enzyme used is immobilised on a water-insoluble support and in that this immobilised enzyme is separated from the biological liquid and washed, before being incubated with the substrate solution in step (2).

2. Process according to claim 1, characterised in that the water-insoluble support is a cross-linked poly(N,N-dimethylacrylamide) resin.

3. Process according to claim 2, characterised in that the immobilisation is effected by attaching the enzyme to the poly(N,N-dimethylacrylamide) resin by a covalent bond.

4. Process according to any of claims 1 to 3, characterised in that the steps (2) and (3) are carried out simultaneously.

5. Process according to any of claims 1 to 4, characterised in that the biological liquid is selected from milk, serum, urine, blood, saliva, meat extracts and fermentation liquids.

6. Process according to any of claims 1 to 5, characterised in that the antibiotic to be determined is selected from benzylpenicillin, ampicillin, phenoxymethylpenicillin, carbenicillin, methicillin, oxacillin, cloxacillin, cephalosporin C, cephaloglycin, cephalothin and cephalexin.

7. Process according to any of claims 1 to 6, characterised in that the substrate is selected from $N^{alpha}$, $N^{epsilon}$-diacetyl-L-lysyl-D-alanyl-D-alanine or $N^{alpha}$-acetyl-L-lysyl-D-alanyl-D-alanine.

8. Process according to any of claims 1 to 7, characterised in that the amount of D-alanine is determined by incubation of mixture obtained in step (2), on the one hand, with a D-aminoacid oxidase, which catalyses the oxidation of the D-alanine to pyruvic acid with the simultaneous formation of hydrogen peroxide, and, on the other and, with a peroxidase and a redox indicator, the latter being oxidised by the hydrogen peroxide formed, by means of the peroxidase, to produce a coloration the intensity of which is a function of the amount of D-alanine.

9. Test set for the determination of antibiotics containing a beta-lactam nucleus in a biological liquid comprising :

(1) a definite amount of soluble D-alanyl-D-alanine-carboxypeptidase produced by Actinomadura R 39 bearing the strain number I-127 at the C.N.C.M., the said enzyme being immobilised on a water-insoluble support ;

(2) a definite amount of substrate ;

(3) reagents allowing the determination of the D-alanine ; and

(4) if appropriate, a standard with which the results of tests carried out with reagents (1), (2) and (3) may be compared.

10. Test set according to claim 9, characterised in that the water-insoluble support is a cross-linked poly(N,N-dimethylacrylamide) resin.

11. Test set according to any of claims 9 and 10, characterised in that the substrate is selected from $N^{alpha}$, $N^{epsilon}$-diacetyl-L-lysyl-D-alanyl-D-alanine or $N^{alpha}$-acetyl-L-lysyl-D-alanyl-D-alanine.

12. Test set according to any of claims 9 to 11, characterised in that the reagents (3) comprise a) a D-aminoacid oxidase, b) a peroxidase and c) a redox indicator.

**Patentansprüche**

1. Enzymatisches Verfahren zur Bestimmung von einen Beta-Laktamkern enthaltende Antibiotika in einer biologischen Flüssigkeit, das die folgenden Stufen enthält :

(1) Inkubation der Flüssigkeit mit der löslichen D-Alanyl-D-alanin-carboxypeptidase, hergestellt von Actinomadura R 39 mit der Stammnummer I-127 bei der C.N.C.M., wobei die Inkubation unter Bedingungen durchgeführt wird, die die Reaktion des gegebenenfalls in der Flüssigkeit vorhandenen Beta-Laktamantibiotikums mit dem Enzym zur Bildung eines inaktiven und merklich irreversiblen, äquimolekularen Enzym-Antibiotikumkomplexes erlauben,

(2) Inkubation des am Ende der Stufe (1) erhaltenen Produkts mit einer Substratlösung unter Bedingungen, die die Hydrolyse des Substrates durch das Enzym ermöglichen zur Bildung einer D-Alanin-Menge, die der restlichen enzymatischen Aktivität entspricht,

(3) Bestimmung der in Stufe (2) gebildeten Menge an D-Alanin,

(4) Vergleich der Bestimmung der Stufe (3) mit einem Standard um die Konzentration des Antibiotikums in der biologischen Flüssigkeit zu erhalten,

dadurch gekennzeichnet, dass das eingesetzte Enzym auf einem wasserunlöslichen Träger immobilisiert wird, und, dass dieses immobilisierte Enzym von der biologischen Flüssigkeit abgetrennt und gewaschen wird, bevor es mit der Substratlösung von Stufe (2) inkubiert wird.

2. Verfahren nach Anspruch 1, dadurch gekennzeichnet, dass der wasserrunlösliche Träger ein vernetztes Poly(N,N-dimethylacrylamid)-Harz ist.

3. Verfahren nach Anspruch 2, dadurch gekennzeichnet, dass die Immobilisierung durch Bindung des Enzyms auf dem Poly(N,N-dimethylacrylamid)-Harz mittels einer kovalenten Bindung hergestellt wird.

4. Verfahren nach irgendeinem der Ansprüche 1 bis 3, dadurch gekennzeichnet, dass die Stufen (2) und (3) gleichzeitig durchgeführt werden.

5. Verfahren nach irgendeinem der Ansprüche 1 bis 4, dadurch gekennzeichnet, dass die biologische Flüssigkeit ausgewählt wird aus Milch, Serum, Urin, Blut, Speichel, Extrakten von Fleisch und Flüssigkeiten der Gärung.

6. Verfahren nach irgendeinem der Ansprüche 1 bis 5, dadurch gekennzeichnet, dass das zu bestimmende Antibiotikum ausgewählt wird aus Benzylpenicillin, Ampicillin, Phenoxymethylpenicillin, Carbenicillin, Methicillin, Oxacillin, Cloxacillin, Cephalosporin C, Cefaloglycin, Cefalotin und Cefalexin.

7. Verfahren nach irgendeinem der Ansprüche 1 bis 6, dadurch gekennzeichnet, dass das Substrat ausgewählt wird aus $N^{alpha}$, $N^{epsilon}$-Diacetyl-L-lysyl-D-alanyl-D-alanin oder $N^{alpha}$-Acetyl-L-lysyl-D-alanyl-D-alanin.

8. Verfahren nach irgendeinem der Ansprüche 1 bis 7, dadurch gekennzeichnet, dass die D-Alanin-Menge durch Inkubation der von Stufe (2) erhaltenen Mischung mit einerseits einer D-Aminosäureoxydase, die die Oxidation von D-Alanin zu Brenztraubensäure katalysiert mit gleichzeitiger Bildung von Wasserstoffperoxid und andererseits einer Peroxydase und einem Redoxindikator, welcher durch das gebildete Wasserstoffperoxid mit Hilfe der Peroxydase oxidiert wird zur Erzeugung einer Färbung, deren Intensität eine Funktion der Menge des D-Alanins sit, bestimmt wird.

9. Testbesteck zur Bestimmung von einen Beta-Laktamkern enthaltende Antibiotika in einer biologischen Flüssigkeit, enthaltend :

(1) eine bestimmte Menge der löslichen D-Alanyl-D-alanin-carboxypeptidase, hergestellt von Actinomadura R 39 mit der Stammnummer I-127 bei der C.N.C.M., wobei das Enzym auf einem wasserunlöslichen Träger immobilisiert ist,

(2) eine bestimmte Substratmenge,

(3) die Reagenzien, die die Bestimmung von D-Alanin erlauben, und

(4) gegebenenfalls einen Standard, mit dem die mit den Reagenzien (1), (2), und (3) erhaltenen Ergebnisse verglichen werden können.

10. Testbesteck nach Anspruch 9, dadurch gekennzeichnet, dass der wasserunlösliche Träger ein vernetztes Poly(N,N-dimethylacrylamid)-Harz ist.

11. Testbesteck nach irgendeinem der Ansprüche 9 und 10, dadurch gekennzeichnet, dass das Substrat ausgewählt ist aus $N^{alpha}$, $N^{epsilon}$-Diacetyl-L-lysyl-D-alanyl-D-alanin oder $N^{alpha}$-Acetyl-L-lysyl-D-alanyl-D-alanin.

12. Testbesteck nach irgendeinem der Ansprüche 9 bis 11, dadurch gekennzeichnet, dass die Reagenzien (3) enthalten a) eine D-Aminosäureoxydase, b) eine Peroxydase und c) einen Redoxindikator.

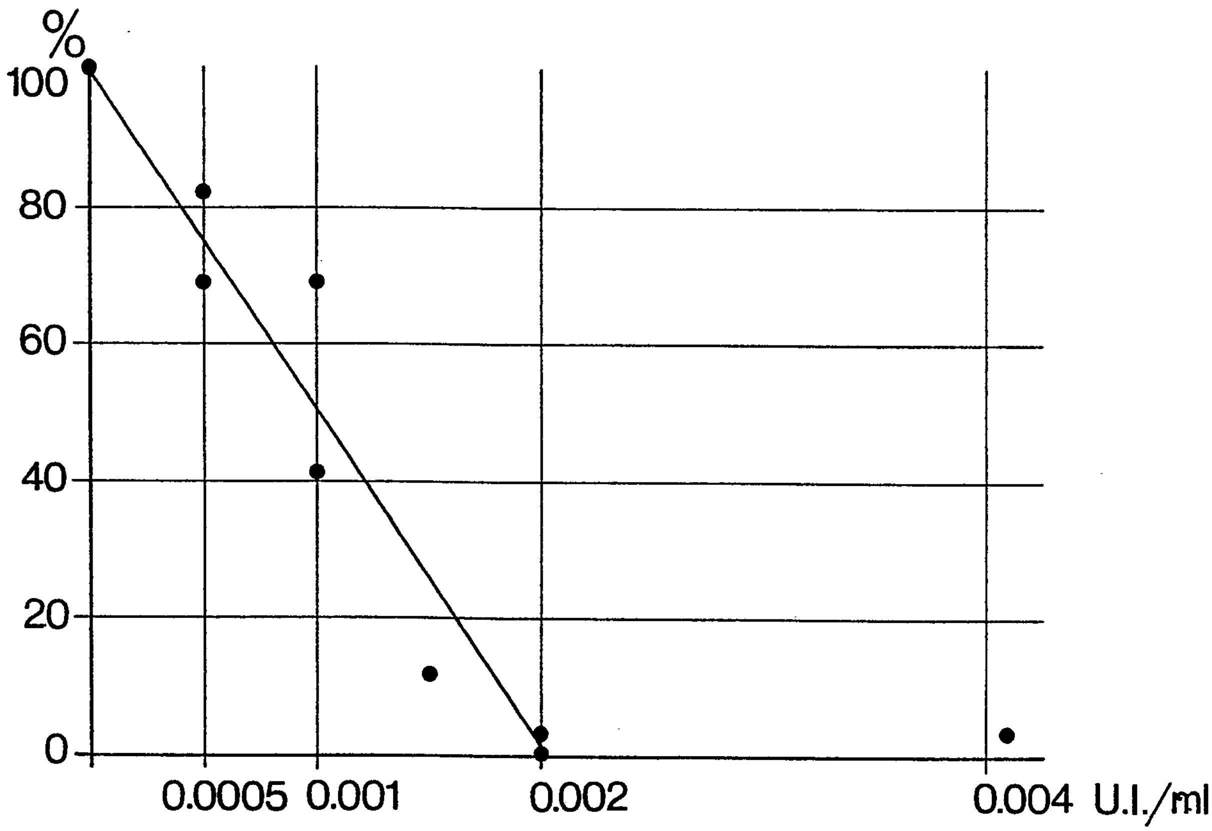
%
100
80
60
40
20
0
0.0005
0.001
0.002
0.004
U.I./ml